# EUROPEAN PATENT SPECIFICATION

(11) **EP 4 129 373 B1**
(45) Date of publication and mention of the grant of the patent: **16.07.2025**
(21) Application number: 21720549.1
(22) Date of filing: 26.03.2021
(51) Int. Cl.: A61M 16/00, A61M 16/08, A61M 16/20, A61M 16/10, A61M 16/16

(54) **INTELLIGENT DISTRIBUTION MODULE FOR VENTILATORS**
INTELLIGENTES VERTEILERMODUL FÜR VENTILATOREN
MODULE DE DISTRIBUTION INTELLIGENTE POUR DES RESPIRATEURS

(30) Priority: 29.03.2020 ES 202030564 U
(43) Date of publication of application: 08.02.2023
(73) Proprietor: Proton New Energy Future, S.L., 08304 Mataró (Barcelona) (ES)
(72) Inventor: CREIXELL SANS, Jordi, 08304 MATARÓ (Barcelona) (ES); JOVE PERALTA, Miquel, 08304 MATARÓ (Barcelona) (ES)
(74) Representative: Herrero & Asociados, S.L.
(86) International application number: PCT/ES2021/070209
(87) International publication number: WO 2021/198544

(56) References cited:
- EP-A2- 0 097 060
- EP-A2- 0 118 850
- WO-A1-2006/067822
- WO-A1-2020/037361
- WO-A2-2011/060204
- CN-A- 105 797 249
- CN-A- 110 292 695
- CN-U- 212 439 636
- ES-U- 1 259 300
- RU-C1- 2 745 261
- US-A- 5 964 220
- US-A1- 2008 295 839
- US-A1- 2019 275 273
- US-B1- 6 474 334
- US-B2- 10 471 229
- US-B2- 8 640 703

## Description

### Field of the art

The art that incorporates the new product is included in the health sector. The object of the present invention is an intelligent distribution module attached to a ventilator, in order to expand the use capacity of up to three people per ventilator.

### Background of the invention

An artificial ventilator can be defined as a machine designed to move air into the lungs, in order to replace the breathing mechanism of a patient who cannot physically inspire or breathe insufficiently. Conventionally artificial ventilators or medical ventilators are used for a single patient.

A ventilator consists of an air and oxygen intake turbine, a compressor and an air adjustment system, a breathable gas tank, a programmable breathable air management system, including a set of valves and tubes, and a disposable or reusable "patient circuit". The compressed breathable air tank is pneumatically supplied to provide the patient with a mixture of air and oxygen.

Currently there is no ventilator that allows treating more than one patient simultaneously using the same device. Artificial respiration equipment is used in the intensive care units of hospitals. Due to the high cost and the space occupied, hospitals have few units to attend to patients and consequently, there is low capacity for patient care.

ES1259300U discloses an intelligent distribution module, to be attached to a respirator, comprising a pressurized reservoir of breathable air and inlet and outlet conductors or tubes.

WO2020037361A1 discloses methods and apparatus that involve generation of an anti-infection therapy. The method/apparatus may include a controller controlling setting of a respiratory flow therapy device for the therapy.

US2008295839A1 discloses a ventilator for use by a clinician in supporting a patient presenting pulmonary distress. A controller module with a touch-screen display operates a positive or negative pressure gas source that communicates with the intubated or negative pressure configured patient through valved supply and exhaust ports.

RU2745261C1 discloses a complex for the centralized distribution of the pressure of the respiratory mixture for simultaneous and individual artificial ventilation of the lungs (avl) to a group of patients and an individual gas distribution unit providing an individual patient with individual avl with the help of the complex.

WO2006067822A1 discloses an aspiratory circuit connected with an inspiratory-expiratory line of a life support machine, which separates at the beginning the gas emission for both lungs.

EP0118850A2 discloses an apparatus comprises one respirator coupled with the lungs by means of a double-lumen tracheal tube (through monitoring-controlling means, containing two parallel inspiratory paths and two parallel expiratory paths.

US2019275273A1 discloses ventilators that provide superior air-oxygen mixing and gas delivery. The ventilators that supply a gas mixture to the lungs of a subject. The gas mixture comprises a first gas (e.g., oxygen) and a second gas (e.g., ambient air).

US8640703B2 discloses a flexible patient cassette that can be optimized for different types of breathing circuits, different drive circuits and different patient categories has a first inlet arranged to be connected to a drive circuit, and at least a second inlet arranged to be connected to a patient connector, the first and second inlets being pneumatically connected with each other through a gas conducting passage constituting a patient circuit having a certain volume.

US10471229B2 discloses a patient transfer device is configured for use with at least one host device which is capable of providing respiratory support to a patient. The patient transfer device includes an inspiratory port and an expiratory port which are configured to pneumatically connect to the host.

### Description of the invention

The inventor of the present application has developed a new intelligent distribution module, which allows a ventilator to be used for one, two or three patients simultaneously. The intelligent distribution module has been designed to adapt to today's ventilators, using the ventilator's air outlet as input to the intelligent distribution module and with up to three air outlets to, as mentioned above, supply up to three patients simultaneously.

The intelligent distribution module according to the present invention is defined in claim 1. Additional features of the module of the present invention are defined in the dependent claims.

The inventor of the present application has designed a new intelligent distribution module that is inserted between the ventilator and the patient tube system, whose main functionalities are:
i) Each of the patients in the group using the intelligent distribution module receives the appropriate air volume and pressure in each inspiration wave.
ii) Each of the patients in the group using the intelligent distribution module receives uncontaminated air.

Furthermore, a method of visual and graphical control of the operation of the intelligent distribution module is also provided (but not claimed), including alarms to ensure that the values are, at all times, within the range previously set by the doctor.

In accordance with the foregoing, a ventilator programmed in non-invasive ventilation (NIV) mode and/or in invasive ventilation mode by tracheal entry consists of the following parts:
i) Pressurized breathable air tank. It consists of an air accumulator that is fed by the fan whose pressure is constantly measured by the pressure sensor built into the tank.
ii) Internal pressure sensor. It consists of a sensor whose functionality is to constantly measure the internal pressure and communicate the parameters to the measurement module.
iii) Air flow sensors. It consists of an air flow sensor for each of the outlets that feed the patient, whose functionality is to continuously measure the air to ensure that the inspiration point is appropriate for the patient and that it is adjusted to the values entered by the healthcare provider.
iv) Pressure sensors. It consists of a pressure sensor for each of the outputs that feed the patient, whose functionality is to measure the individual pressure, to obtain data such as PEEP, pressure in real time (cmH20) or maximum pressure.
v) Manual regulation or electrical regulation valves. It consists of manual or regulation valves whose functionality is to regulate the inspiration point for each patient. It allows the healthcare provider to distribute the air between the patients in a regulated way. vi) Antibacterial inspiration filters. It consists of an antibacterial filter per patient to ensure that the air that the patient breathes in is free from bacteria and viruses.
vii) Humidifiers. It consists of one humidifier per patient to regulate the humidity of the inspiration air.
viii) Check valves. It consists of a non-return valve located in each individualized circuit per patient, whose function is to ensure that air cross contamination is not generated
ix) 22 millimeter ISO extension tubing.
x) Distributor controller. It consists of an HMI touch screen that allows interaction between the distribution module and the healthcare provider, and an analog measurement module that collects the sensor readings, digitizes them and sends them to the HMI touch screen in order to coordinate all the intelligent distribution module.

In addition, during the breathing sequence, the patient performs an exhalation outlet. This expiration outlet, already known in the art, consists of an expiration tube, which expels the used air to the environment, using a condensate vial and a bacterial expiration filter.

### Brief description of the drawings

To complement the description that is being made and in order to help a better understanding of the features of the invention, a set of drawings is attached as an integral part of said description, in which, with an illustrative and non-limiting nature, the following has been represented:
FIG. 1 shows a front view of the intelligent distribution module, according to a first embodiment according to the invention as claimed;
FIG. 2 shows a top view of the intelligent distribution module, according to the first embodiment;
FIG. 3 shows a front view of the intelligent distribution module, according to a second embodiment according to the invention as claimed;
FIG. 4 shows a front view of the intelligent distribution module, according to a third embodiment according to the invention as claimed; and
FIG. 5 shows a top view of the intelligent distribution module, according to the third embodiment.

### Preferred embodiments of the invention

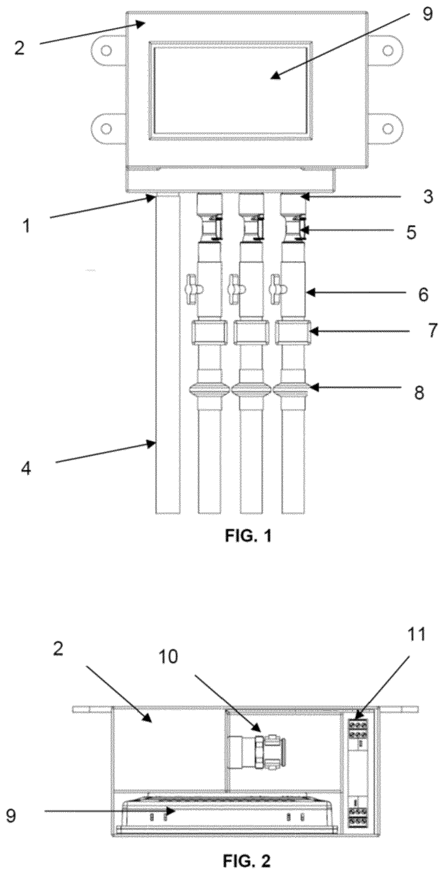

In the following, the system according to the present invention is described with reference to the attached figures. Specifically, Figures 1 and 2 show a first embodiment.

An intelligent distribution module attached to a ventilator is shown, whose purpose is to assist patients who require artificial respiration. Outlet tubes (3) of the distribution module can supply up to three patients simultaneously from a single ventilator, by virtue of the air management provided by the present invention.

The module has an air inlet tube (1, 4) that comes from a conventional fan. The operation of the ventilator known in the art may utilize the forced inspiration mode, in which the ventilator generates inspiration air waves of programmable volume and pressure on a programmable time basis.

A pressurized breathable air tank (2) is an air accumulator and receives the air wave caused by the ventilator. The pressure of the pressurized tank (2) can be measured with a pressure sensor (10), the working range of which is from 0 to 7 bars, and feeds, preferably three air outlet tubes (3) each one of the three tubes (3) including inside: an air flow sensor (5), a manual valve (6), preferably manual ISO 22 millimeters, a pressure sensor (13) whose working range is from -250 to +250 SLPM, an antibacterial filter (7), a humidifier (8), a check valve and finally, and depending on the method, a non-invasive set with a mask or an invasive set with tracheal inlet. In addition, the intelligent distribution module contains a control system designed to incorporate active regulation of the three outlet tubes by means of electrically actuated valves, and automatically adjust the inspiration point for each patient.

The control system is composed of an HMI touch screen (9) in which the inspiration point differences between the patients in the group are entered, the pressure and the evolution of the inspiration points of the three patients are continuously monitored; an analog-digital converter measurement module (11) that reads the measurements, whose inputs are analog and converts them into digital outputs so that the HMI touch screen (9) is able to process the data and display them on the screen (9).

Based on the values obtained from the monitoring, the control system allows the programming of display modes and alarms, to indicate to the healthcare provider if there is a variation in the inspiration point of one or more patients, outside the parameters programmed at the beginning of the therapy.

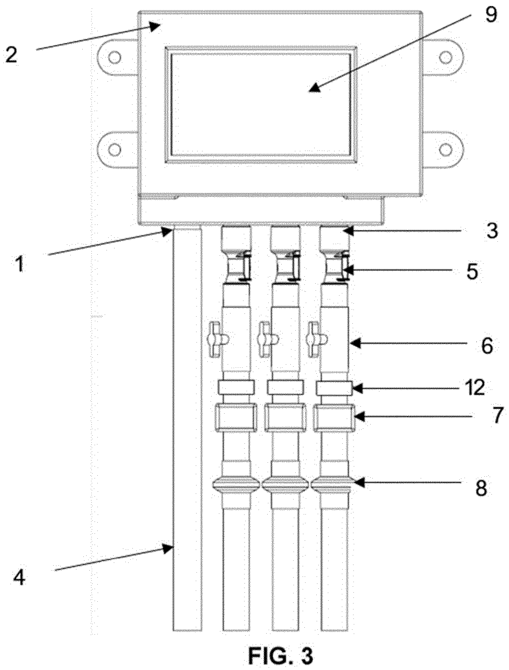

In figure 3 a second embodiment of the intelligent distribution module according to the present invention is shown.

The only difference compared to the previous embodiment is that each outlet tube (2) comprises a pressure sensor (12) for the patient, which is located after each valve (6).

The working pressure range is preferably -250 to +250 SLPM (standard liters per minute). Furthermore, this pressure sensor (12) is individualized for each of the patients, the common pressure sensor (10), shown in Figure 2, not being necessary.

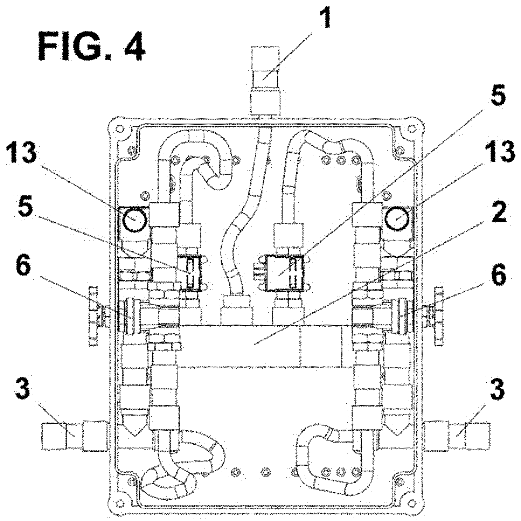

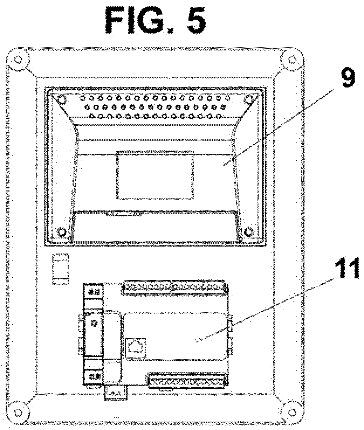

A third embodiment of the distribution module according to the present invention is shown in Figures 4 and 5.

Although the configuration is different from the first embodiment shown in Figures 1 and 2, the components that it includes are the same, the same reference numerals being used to identify the same components.

The intelligent distribution module is designed based on the following parameters:
- Air wave volume from 2 mL to 315 mL
- Respiratory rate 1 / min to 150 / min
- Inspiration time 0.2 to 8 seconds
- Maximum flow 1 L / min to 30 L / min

## Claims

1. Intelligent distribution module, to be attached to a ventilator, the intelligent distribution module comprising a pressurized breathable air tank (2) and at least one inlet tube (1) and at least one outlet tube (3),
the intelligent distribution module also comprising a control system, including an HMI human interface central unit,
**characterized in that** the intelligent distribution module includes a set of components comprising:
- the or each outlet tube (3) is equipped with one or more air flow sensors (5), one or more manual valves (6) and one or more antibacterial filters (7).

2. Intelligent distribution module according to claim 1, **characterized in that** the control system comprises an HMI screen (9) and an analog-digital converter module (11), which can manage one, two or three air outlet tubes (3) simultaneously.

3. Intelligent distribution module according to claim 1, **characterized in that** it includes one or more pressure sensors (10) in the pressurized breathable air tank (2).

4. Intelligent distribution module according to claim 1, **characterized in that** the one or more air flow sensors (5) are proximal and/or bidirectional.

5. Intelligent distribution module according to claim 1, **characterized in that** the one or more manual valves (6) allow the passage of air flow from 0% to 100%.

6. Intelligent distribution module according to claim 5, **characterized in that** the one or more manual valves (6) are electric valves.

7. Intelligent distribution module according to claim 1, **characterized in that** it comprises a humidifier (8) in one, two or three outlet tubes (3) of the intelligent distribution module, located between the or each antibacterial filter (7) and an air inlet pathway to the patient.

## Patentansprüche

1. Intelligentes Verteilermodul zur Anbringung an einem Beatmungsgerät, wobei das intelligente Verteilermodul einen druckbeaufschlagten Atemlufttank (2) sowie mindestens einen Einlassschlauch (1) und mindestens einen Auslassschlauch (3) umfasst,
wobei das intelligente Verteilermodul des Weiteren ein Steuersystem mit einer Mensch-Maschine-Schnittstelle-(MMS)-Zentraleinheit umfasst,
**dadurch gekennzeichnet, dass** das intelligente Verteilermodul eine Reihe von Komponenten aufweist, Folgendes umfassend:
- Der bzw. jeder Auslassschlauch (3) ist mit einem oder mehreren Luftstromsensoren (5), einem oder mehreren manuellen Ventilen (6) und einem oder mehreren antibakteriellen Filtern (7) ausgestattet.

2. Intelligentes Verteilermodul nach Anspruch 1, **dadurch gekennzeichnet, dass** das Steuersystem einen MMS-Bildschirm (9) und ein Analog-Digital-Wandlermodul (11) umfasst, das einen, zwei oder drei Luftauslassschläuche (3) gleichzeitig verwalten kann.

3. Intelligentes Verteilermodul nach Anspruch 1, **dadurch gekennzeichnet, dass** es einen oder mehrere Drucksensoren (10) in dem druckbeaufschlagten Atemlufttank (2) umfasst.

4. Intelligentes Verteilermodul nach Anspruch 1, **dadurch gekennzeichnet, dass** der eine oder die mehreren Luftstromsensoren (5) proximal und/oder bidirektional ausgebildet sind.

5. Intelligentes Verteilermodul nach Anspruch 1, **dadurch gekennzeichnet, dass** das eine oder die mehreren manuellen Ventile (6) einen Durchtritt eines Luftstroms von 0 % bis 100 % zulassen.

6. Intelligentes Verteilermodul nach Anspruch 5, **dadurch gekennzeichnet, dass** das eine oder die mehreren manuellen Ventile (6) als elektrische Ventile ausgebildet sind.

7. Intelligentes Verteilermodul nach Anspruch 1, **dadurch gekennzeichnet, dass** es eine Befeuchtungseinrichtung (8) in einem, zwei oder drei Auslassschläuchen (3) des intelligenten Verteilermoduls umfasst, die zwischen dem bzw. jedem antibakteriellen Filter (7) und einem Lufteinlassweg zu dem Patienten angeordnet ist.

## Revendications

1. Module de distribution intelligent, à fixer sur un ventilateur, le module de distribution intelligent comprenant un réservoir d'air respirable pressurisé (2) et au moins un tube d'entrée (1) et au moins un tube de sortie (3),
le module de distribution intelligent comprenant également un système de contrôle, incluant une unité centrale d'interface homme IHM,
**caractérisé en ce que** le module de distribution intelligent inclut un ensemble de composants comprenant :
- le ou chaque tube de sortie (3) est équipé d'un ou plusieurs capteurs de débit d'air (5), une ou plusieurs vannes manuelles (6) et un ou plusieurs filtres antibactériens (7).

2. Module de distribution intelligent selon la revendication 1, **caractérisé en ce que** le système de contrôle comprend un écran d'IHM (9) et un module convertisseur analogique-numérique (11), qui peut gérer un, deux ou trois tubes de sortie d'air (3) simultanément.

3. Module de distribution intelligent selon la revendication 1, **caractérisé en ce qu'**il inclut un ou plusieurs capteurs de pression (10) dans le réservoir d'air respirable pressurisé (2).

4. Module de distribution intelligent selon la revendication 1, **caractérisé en ce que** le ou les capteurs de débit d'air (5) sont proximaux et/ou bidirectionnels.

5. Module de distribution intelligent selon la revendication 1, **caractérisé en ce que** la ou les vannes manuelles (6) permettent le passage de flux d'air de 0% à 100%.

6. Module de distribution intelligent selon la revendication 5, **caractérisé en ce que** la ou les vannes manuelles (6) sont des vannes électriques.

7. Module de distribution intelligent selon la revendication 1, **caractérisé en ce qu'**il comprend un humidificateur (8) dans un, deux ou trois tubes de sortie (3) du module de distribution intelligent, situés entre le ou chaque filtre antibactérien (7) et un chemin d'entrée d'air jusqu'au patient.
